# EUROPEAN PATENT SPECIFICATION

(11) **EP 3 555 121 B1**
(45) Date of publication and mention of the grant of the patent: **22.09.2021**
(21) Application number: 17837873.3
(22) Date of filing: 18.12.2017
(51) Int. Cl.: A01N 37/46, A23K 10/00, A23L 3/3571, A61P 31/04, C12N 9/36, A61K 38/00

(54) **NOVEL ENDOLYSIN**
NEUARTIGES ENDOLYSIN
NOUVELLE ENDOLYSINE

(30) Priority: 16.12.2016 EP 16204770
(43) Date of publication of application: 23.10.2019
(73) Proprietor: Universidade do Minho, 4700-320 Braga (PT)
(72) Inventor: MENDES DE OLIVEIRA, Hugo Alexandre, 4715-595 Braga (PT); VALENTE DE RODRIGUES AZEREDO, Joana Cecilia, 4700-752 Braga (PT)
(74) Representative: Rückerl, Florian
(86) International application number: PCT/EP2017/083326
(87) International publication number: WO 2018/109229

(56) References cited:
- WO-A1-2011/134998
- WO-A1-2012/059545
- WO-A2-2010/023207
- OLIVEIRA HUGO ET AL: "Characterization and genome sequencing of aCitrobacter freundiiphage CfP1 harboring a lysin active against multidrug-resistant isolates", APPLIED MICROBIOLOGY AND BIOTECHNOLOGY, SPRINGER, DE, vol. 100, no. 24, 28 September 2016 (2016-09-28), pages 10543-10553, XP036105346, ISSN: 0175-7598, DOI: 10.1007/S00253-016-7858-0 [retrieved on 2016-09-28]
- Maarten Walmagh: "ARENBERG DOCTORAL SCHOOL FACULTEIT BIO-INGENIEURSWETENSCHAPPEN DEPARTEMENT BIOSYSTEMEN DEVELOPMENT AND EVALUATION OF ENGINEERED BACTERIOPHAGE ENDOLYSINS FOR INACTIVATION OF GRAM-NEGATIVE BACTERIA", , 1 January 2013 (2013-01-01), XP055354016, Retrieved from the Internet: URL:https://lirias.kuleuven.be/bitstream/1 23456789/371706/1/Doctoral+Thesis+Maarten+ Walmagh.pdf [retrieved on 2017-03-13]

## Description

### FIELD OF THE INVENTION

The present invention relates to a polypeptide with endolysin activity and comprising an amino acid sequences according to SEQ ID No. 1 or fragments or derivatives thereof wherein the derivative is an amino acid sequence having a substitution of 1, 2, 3, 4, or 5 amino acids in the amino acid sequence of SEQ ID No. 1 and/or 3, and wherein the fragment comprises an amino acid sequence according to SEQ ID No. 3, SEQ ID No. 5 or SEQ ID No. 6. Moreover, the present invention relates to nucleic acid molecules encoding said polypeptide, vectors comprising said nucleic acid molecules and host cells comprising either said nucleic acid molecules or said vectors. In addition, the present invention relates to said polypeptide for use as a medicament, in particular for the treatment or prevention of Gram-negative bacterial infections, as diagnostic means, as cosmetic substance or as sanitizing agent. The present invention also relates to the use of said polypeptide for the treatment or prevention of bacterial contamination, particularly of Gram-negative contamination, of foodstuff, of food processing equipment, of food processing plants, of surfaces coming into contact with foodstuff, of medical devices, of surfaces in hospitals and doctor's offices. Furthermore, the present invention relates to a pharmaceutical composition comprising said polypeptide.

### BACKGROUND OF THE INVENTION

Life-threatening human infectious diseases caused by bacterial pathogens are now reemerging in part due to the increased resistance to antibiotics. Gram-negative bacteria correspond to a prominent part of drug resistant pathogens and have become a major public health threat leading to an increasing morbidity and mortality caused by infection diseases. Many efforts have been put forward but even so the rate of multidrug-resistant Gram-negative bacteria is still rising throughout the world and has become a growing problem. There is a scarcity or even inexistence of new active antibiotics specifically developed for multidrug-resistant Gram-negative bacteria in contrast with what happened with Gram-positive bacteria and consequently there are now a growing number of Gram-negative organisms isolated in clinical samples and environments classified as pan-drug resistant isolates, i.e., bacteria resistant to all available antibiotics.

The Gram-negative cell wall is constituted by an outer membrane which is a major obstacle to most antimicrobials. With a few exceptions, the outer membrane consists of an asymmetric membrane of phospholipids (inner leaflet), lipopolysaccharides (LPS) (outer leaflet) and proteins, like porins, serving as defusing channels, and lipoproteins, covalently bound to the peptidoglycan and anchored to the inner leaflet. The LPS is the major component of the outer membrane and provides the bacterium a permeability barrier for many external agents. The LPS is an extremely diverse structure that can be modified in response to prevailing environmental conditions (Nikaido, 2003). Three different regions can be distinguished according to their chemical structure, biosynthesis, genetics and function: i) the lipid A component, ii) the core region and iii) the O-antigen, also known as O-specific polysaccharides or O-side chains. The LPS may be presented in a rough (R-type, without O-antigen), smooth (S-type, with O-antigen) or a mixture (SR-type). The overall outer membrane integrity is maintained by two main stabilizing forces, the LPS cation-binding sites (ionic interactions) and the dense hydrophobic stacking of the Lipid A acyl chain groups (hydrophobic interactions), and act as an efficient diffusion barrier function as a natural barrier against antimicrobials. Thus, there is a need for new antimicrobial agents active against Gram-negative bacteria.

Endolysins used as 'enzybiotics' - a hybrid term of 'enzymes' and 'antibiotics' - perfectly met this need. Endolysins are specialized peptidoglycan-degrading enzymes encoded by all double stranded DNA bacteriophages (Young et al., Trends Microbiol. 2000 Mar;8(3):120-128). Endolysin-mediated action is a coordinated and regulated event by other proteins to degrade and compromise the bacterial peptidoglycan (PG) layer at the end of the bacteriophage replication cycle. Depending on their activity and the bonds that they hydrolyse, endolysins can be divided into different classes: i) Glycosidases that cleave the glycan component at the reducing end of N-acetylglucosamine (GlcNAc) or at the reducing end of N-acetylmuramic acid (MurNAc); ii) N-acetyl-β-D-muramidases (often called lysozymes or muramidases) that share the same glycan target as the lytic transglycosidases, but deliver different end products; iii) amidases that catalyze the hydrolysis of the critical amide bond between MurNAc and the L-alanine, separating the glycan strand from the stem peptide; and iv) distinct classes of endopeptidases and carboxypeptidases that attack the LD- and DD-bonds in the stem peptides that crosslink the cell wall.

While the potential of endolysins has been known for a long time, their antibacterial use was overshadowed by the success and dominance of antibiotics. Only due to the alarming emergence of antibiotic resistant bacteria, scientific community and business players have revived their interest in endolysins as an alternative therapy. In 2001 the first endolysin study revealed a great capacity to destroy group A, C and E streptococci in seconds leaving 14 other commensal streptococci unharmed. This unique ability of endolysins to rapidly cleave PG in a species-specific manner when added exogenously, instigated researchers to isolate a huge number of endolysins to successfully target other Gram-positive pathogens. However, endolysins attacking Gram-negative cells, have received little attention mostly due to the presence of a highly protective outer membrane which the endolysins by itself cannot destroy in order to be able to act on the PG. As a result, an endolysin-based technology to target Gram-negative cells such as hospital and foodborne pathogens (e.g. *Salmonella, Escherichia coli, Pseudomonas* spp., *Acinetobacter* spp.) is perhaps one of the most important challenges in endolysin therapy today.

### SUMMARY OF THE INVENTION

The present invention relates to a novel polypeptide with peptidoglycan degrading activity, isolated from *Citrobacter koseri* phage CkP1, with antimicrobial activity against Gram-negative bacteria.

A first object of the present invention provides a polypeptide having endolysin activity comprising an amino acid sequence according to SEQ ID No. 1 or a fragment or derivative thereof, wherein the derivative is an amino acid sequence having a substitution of 1, 2, 3, 4, or 5 amino acids in the amino acid sequence of SEQ ID No. 1 and/or 3, and wherein the fragment comprises an amino acid sequence according to SEQ ID No. 3, SEQ ID No. 5 or SEQ ID No. 6. The polypeptide comprising an amino acid sequence according to SEQ ID No. 1 is preferably encoded by a nucleotide sequence according to SEQ ID No. 2. In a preferred embodiment said fragment comprises an amino acid sequence according to SEQ ID No. 3, which is preferably encoded by nucleotide sequence SEQ ID No. 4. In yet another preferred embodiment, said polypeptide according to the present invention comprises at the N- or C-terminus a peptide stretch having membrane or LPS disrupting activity, in particular a cationic or polycationic peptide. Said polypeptide may additionally comprise a tag, preferably a His6-tag.

A second object of the present invention provides an isolated nucleic acid molecule encoding a polypeptide according to the invention.

A third object of the present invention relates to a vector comprising a nucleic acid molecule according to the present invention.

A fourth object of the present invention relates to a host cell comprising a nucleic acid molecule according to the present invention or a vector according to the present invention.

A fifth object of the present invention relates to the polypeptide according to the present invention for use in a method of treating the human or animal body by surgery or therapy and or for use in a diagnostic method practised on the human or animal body. In particular, the present invention relates to the polypeptide according to the present invention for use in a method of treating or preventing Gram-negative bacterial infections in a subject, such as a human or an animal. The polypeptide according to the present invention can also be used as diagnostic substance. For example, lysis of bacteria upon ex vivo contact with a polypeptide according to the present invention (e.g. in a bacterial culture established from a sample of a subject or from the environment) indicates presence of Gram-negative bacteria.

A sixth object of the present invention relates to the use of a polypeptide according to the present invention as an antimicrobial in food, feed or in cosmetics, or as a disinfecting agent. Another preferred embodiment relates to the use of said polypeptide according to the present invention for the treatment or prevention of Gram-negative bacterial contamination of foodstuff, of food processing equipment, of food processing plants, of (inanimate) surfaces coming into contact with foodstuff, of feed, of feed processing equipment, of feed processing plants, of (inanimate) surfaces coming into contact with feed, of medical devices, of (inanimate) surfaces in hospitals and doctor's offices.

### DETAILED DESCRIPTION

### Legends to the figures

The following figures serve to illustrate the invention.

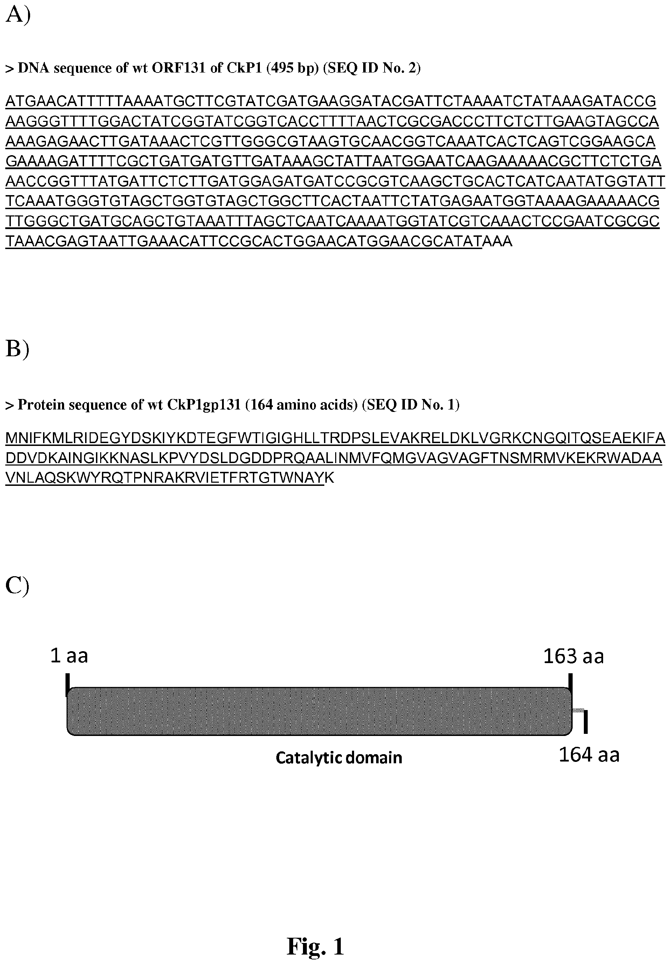

Figure 1. Bioinformatics analysis of the wild type *Citrobacter koseri* endolysin CkP1gp131 using BLASTp and Pfam systems. The DNA and amino acid sequences as they appear in the wild type phage are A) SEQ ID No. 2 and B) SEQ ID No. 1, respectively. The catalytic domain of the lysozyme-like superfamily (amino acids 1- 163, SEQ ID No. 3, underlined) is visualized in the scheme (Fig.1 C) and the sequences (Fig. 1A and B; underline).

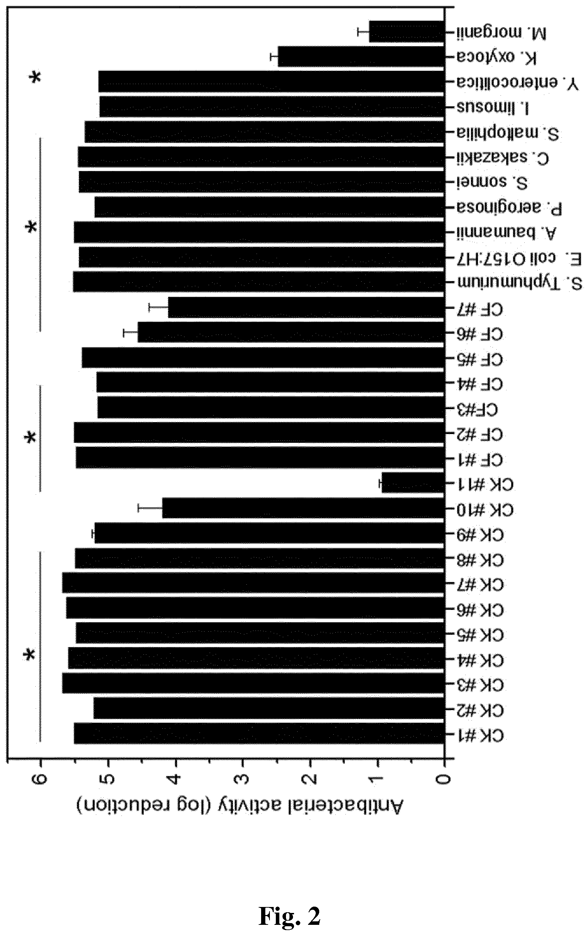

Figure 2. Antibacterial activity of CkP1gp131 against several Gram-negative bacterial pathogens. Exponentially growing cells (≈3x10⁷ CFU/ml) were incubated for 2 hours and at 37°C, with 50 µg/ml (2.4 µM) of CkP1gp131 resuspended in 20 mM HEPES pH 7.0 or with HEPES as a negative control. After incubation, the antibacterial effect was assessed by quantification of the number of CFUs and expressed as relative inactivation in logarithmic units (= log10 (N₀/Nᵢ) with N₀ = number of untreated cells and Nᵢ = number of cells after treatment). Averages ± standard deviations are given for n = 3 repeats. CK - *Citrobacter koseri* strains (#1 to 11); CF - *Citrobacter freundii* strains (#1 to 7); * below the limit of detection (<10 cfu/ml).

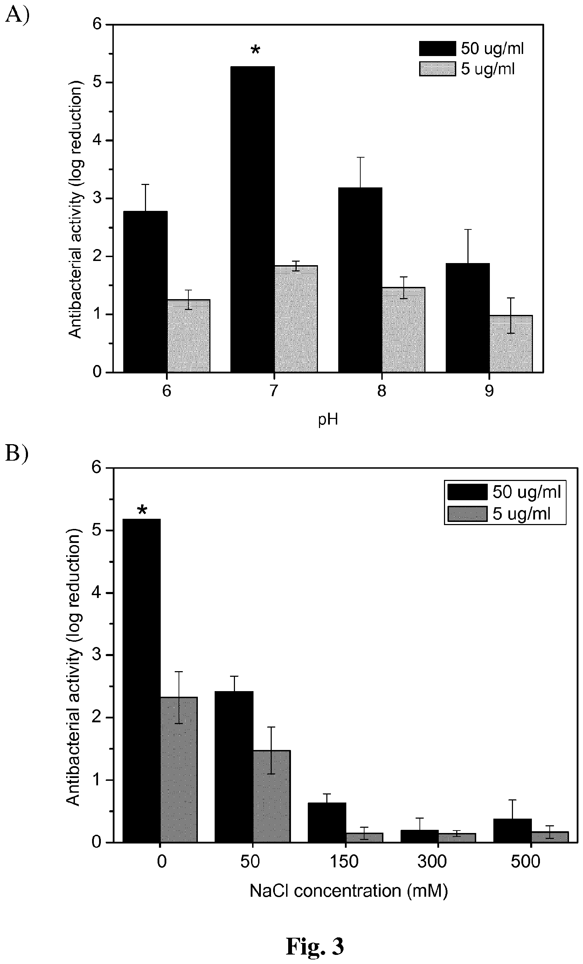

Figure 3. Antibacterial activity of CkP1gp131: A) Activity under different pH values. B) Activity under different ionic strength. Two concentrations of endolysin (5 and 50µg/ml) were tested.
Activity is expressed as relative inactivation in logarithmic units (= log10 (N0/Ni) with N0 = number of untreated cells and Ni = number of cells after treatment). Averages ± standard deviations are given for n = 4 repeats. * below the limit of detection (<10 CFU/ml).

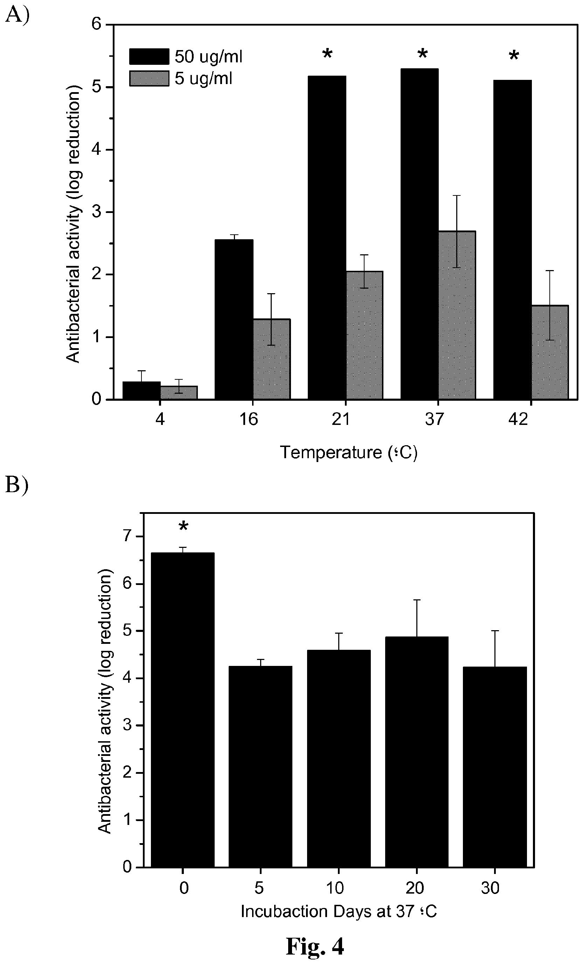

Figure 4. Antibacterial activity of CkP1gp131: A) Activity under different temperatures. Two concentrations of endolysin (5 and 50µg/ml) were tested. Averages ± standard deviations are given for n = 4 repeats. B) Activity over a period of 30 days (50µg/ml). Activity is expressed as relative inactivation in logarithmic units (= log10 (N0/Ni) with N0 = number of untreated cells and Ni = number of cells after treatment). * below the limit of detection (<10 CFU/ml).

### Description

### Definitions:

If appearing herein, the following terms shall have the definitions set out below.

The term "polypeptide" as used herein refers synonymously to the term "protein". The term "polypeptide" as used herein refers to a linear polymer of amino acid residues linked by peptide bonds in a specific sequence. The amino acid residues of a protein may be modified by e.g. covalent attachments of various groups such as carbohydrates and phosphate. Other substances may be more loosely associated with the polypeptide chains, such as heme or lipid, giving rise to the conjugated proteins which are also comprised by the term "polypeptide" as used herein. The various ways in which the polypeptide chains fold have been elucidated, in particular with regard to the presence of alpha helices and beta-pleated sheets. The term "polypeptide" as used herein refers to all four classes of proteins being all-alpha, all-beta, alpha/beta and alpha plus beta.

The term "peptide stretch" as used herein refers to any kind of peptide linked to a protein such as an endolysin. However, a peptide stretch in the meaning of the present invention does not refer to His6-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "peptide stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His6-tag may, depending on the respective pH, also be positively charged, but is used as affinity purification tool as it binds to immobilized divalent cations and is not used as a peptide stretch according to the invention.

The term "peptide stretch" as used herein refers to short polypeptide sequences consisting of from about 2 to 10 about 100 amino acid residues, more preferably from about 4 to about 50 amino acid residues, more preferably from about 5 to about 30 amino acid residues, wherein the amino group of one amino acid residue is linked to the carboxyl group of another amino acid residue by a peptide bond.

The term "endolysin activity" as used herein refers to the activity of hydrolysing bacterial cell walls, in particular by degrading bacterial peptidoglycan such as the peptidoglycan of Gram-negative bacteria.

The term "wild type" or "wt" as used herein refers to the amino acid sequence of the endolysin CkP1gp131 as depicted in SEQ ID No. 1. The nucleic acid sequence encoding the wild type endolysin CkP1gp131 is depicted in SEQ ID No. 2.

The term "substitution" as used herein refers to the presence of an amino acid or nucleic acid residue located at a certain position of the derivative sequence, which is different from the amino acid or nucleic acid residue which is present or absent at the corresponding position in the reference sequence. A derivative of the present invention may exhibit 1, 2, 3, 4, 5, 6, 7, 8, 9, 10, 11, 12, 13, 14, 15, 16, 17, 18, 19, 20, or more of such substitutions. As mentioned above, preferably such substitutions are conservative substitutions.

The term "cell wall" as used herein refers to all components that form the outer cell enclosure of the Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The present invention relates to a new endolysin, isolated from *Citrobacter koseri* phage CkP1, useful in the preparation of novel antibacterial agents against Gram-negative bacteria. More specifically, the present invention relates to a polypeptide comprising an amino acid sequence according to SEQ ID No. 1 or fragments or derivatives thereof, wherein the derivative is an amino acid sequence having a substitution of 1, 2, 3, 4, or 5 amino acids in the amino acid sequence of SEQ ID No. 1 and/or 3, and wherein the fragment comprises an amino acid sequence according to SEQ ID No. 3, SEQ ID No. 5 or SEQ ID No. 6. The polypeptide comprising an amino acid sequence according to SEQ ID No. 1 is preferably encoded by a nucleotide sequence according to SEQ ID No. 2.

The endolysin CkP1gp131 having an amino acid sequence according to SEQ ID No. 1 has a length of 164 amino acids. The enzymatic active domain (EAD) (aa 1 to 163) complies with the lysozyme-like superfamily domain motif with an amino acid sequence according to SEQ ID No. 3 and a nucleotide sequence according to SEQ ID No. 4.

Fragments of SEQ ID No. 1 comprise an amino acid sequence according to SEQ ID No. 3. Other fragments of SEQ ID No. 1 are SEQ ID No. 5 and SEQ ID No. 6 which lack the N-terminal methionine of CkP1gp131 Preferably, a polypeptide according to the present invention comprising a fragment of SEQ ID No. 1 does not comprise the sequence of SEQ ID No. 1. Preferably, a polypeptide according to the present invention comprising a fragment of SEQ ID No. 1 exhibits essentially the lytic activity of the wt CkP1gp131 endolysin (SEQ ID No. 1). Said activity represents preferably about 10, 20, 30, 40, 50, 60, 70, 80, 90, 100, 110, 120, 130, 140, 150, 160, 170, 180, 190 or about 200 % of the activity of the wt CkP1gp131 endolysin.

In some embodiments, the polypeptide of the present invention comprises an amino acid sequence, which is a derivative of the amino acid according to SEQ ID No. 1, wherein the derivative is an amino acid sequence having an amino acid sequence having a substitution of 1, 2, 3, 4, or 5 amino acids in the amino acid sequence of SEQ ID No. 1 and/or 3. Such derivatives may be polypeptides comprising essentially an amino acid sequence according to SEQ ID No. 1, SEQ ID No. 3, SEQ ID No. 5 and/or SEQ ID No. 6 but having additional modifications and/or alterations.

Said derivatives according to the present invention exhibit essentially the lytic activity of the wt CkP1gp131 endolysin (SEQ ID No. 1). Said activity represents preferably about 10, 20, 30, 40, 50, 60, 70, 80, 90, 100, 110, 120, 130, 140, 150, 160, 170, 180, 190 or about 200 % of the activity of the wt CkP1gp131 endolysin.

The activity of polypeptides comprising fragments and derivatives of wt CkP1gp131 endolysin (SEQ ID No. 1) can be measured by assays well known in the art by a person skilled in the art as e.g. the plate lysis assay or the liquid lysis assay which are e.g. described in (Briers et al., J. Biochem. Biophys 20 Methods 70: 531-533, (2007)).

In a preferred embodiment of the present invention the polypeptide according to the present invention comprises additionally a tag such as a His6-tag, Strep-tag, Avi-tag, Myc-tag, Gst-tag, JS-tag, cystein-tag, FLAG-tag or other tags known in the art at the N-terminus or at the C-terminus. In a preferred embodiment of the present invention said tag is linked to the sequence of SEQ ID No. 1, or the fragment and/or derivative thereof, at the C-terminus (e.g. N-tag-SEQ ID No. 1-C). Said tag may be linked to SEQ ID No. 1, or the fragment or derivative thereof over additional amino acid residues. Said additional amino acid residues may consist of at least 1, 2, 3, 4, 5, 6, 7, 8, 9 or 10 additional amino acid residues. In a preferred embodiment of the present invention the tag is linked to SEQ ID No. 1, the fragment or the derivative by the additional amino acid residues Leu-Glu or Lys-Gly. In a preferred embodiment the present invention relates to a polypeptide comprising an amino acid sequence according to SEQ ID No. 7. The polypeptide having an amino acid sequence according to SEQ ID No. 7 comprises in comparison to the polypeptide having an amino acid sequence according to SEQ ID No. 1, respectively, an additional His6-tag linked to the N-terminus of SEQ ID No.1. A polypeptide comprising an amino acid sequence according to SEQ ID No. 7 is preferably encoded by a nucleotide sequence according to SEQ ID No. 8.

The present invention further relates to an isolated nucleic acid molecule encoding a polypeptide according to the present invention. Especially preferred isolated nucleic acid molecules according to the present invention comprise a nucleic acid sequence according to SEQ ID No. 2, SEQ ID No. 4 and/or SEQ ID No. 8. Preferably, the nucleic acid molecule comprises a promotor heterologous to SEQ ID No. 1.

The present invention further relates to a vector comprising the nucleic acid molecule according to the present invention. Said vector may provide for the constitutive or inducible expression of said polypeptide according to the present invention.

The invention also relates to a host cell comprising a polypeptide, nucleic acid and/or vector according to the present invention. The host cell may be a genetically modified suitable host cell which expresses said polypeptide. Said host cell may be a micro-organism such as bacteria or yeast or an animal cell as e.g. a mammalian cell, in particular a human cell. A host cell according to the present invention is not part of a multicellular organism but isolated (e.g., an immortalized cell line etc.). In one embodiment of the present invention the host cell is an *Escherichia coli* cell. The host may be selected due to mere biotechnological reasons, e.g. yield, solubility, costs, etc. but maybe also selected from a medical point of view, e.g. a nonpathological bacteria or yeast or human cells.

Another aspect of the present invention is related to a method for genetically transforming a suitable host cell in order to obtain the expression of the polypeptide according to the present invention, wherein the host cell is genetically modified by the introduction of a nucleic acid according to the present invention encoding said polypeptide according to the present invention into the host cell. Translation and expression of the polypeptide according to the present invention can be obtained by genetic engineering methods well known by the man skilled in the art.

The invention also relates to a method for obtaining a polypeptide according to the present invention from a host cell according to the present invention, such as a genetically modified suitable host cell which expresses said polypeptide, wherein the method comprises isolating the a polypeptide according to the present invention from said host cell.

In a further aspect the present invention relates to a composition, preferably a pharmaceutical composition, comprising a polypeptide according to the present invention and/or a host cell transformed with a nucleic acid molecule or a vector according to the present invention, and further comprising a diluent, excipient or carrier, such as a pharmaceutical acceptable diluent, excipient or carrier.

In a preferred embodiment of the present invention the composition comprises additionally agents permeabilizing the outer membrane of Gram-negative bacteria such metal chelators as e.g. EDTA, TRIS, lactic acid, lactoferrin, polymyxin, citric acid and/or other substances as described e.g. by Vaara (Agents that increase the permeability of the outer membrane. Vaara M. Microbiol Rev. 1992 Sep;56(3):395-441). Also preferred are compositions comprising combinations of the above mentioned permeabilizing agents. Especially preferred is a composition comprising about 10 µM to about 100 mM EDTA, more preferably about 50 µM to about 10 mM EDTA, more preferably about 0.5 mM to about 10 mM EDTA, more 30 preferably about 0.5 mM to about 2 mM EDTA, more preferably about 0.5 mM to about 1 mM EDTA. Also preferred is a composition comprising about 0.5 mM to about 2 mM EDTA, more preferably about 1 mM EDTA and additionally about 10 to about 100 mM TRIS.

The present invention also relates to a polypeptide according to the present invention and/or a host transformed with a nucleic acid comprising a nucleotide sequence encoding a polypeptide according to the present invention for use in a method of treating the human or animal body by surgery or therapy and diagnostic methods practised on the human or animal body. In a further aspect the present invention relates to the use of a polypeptide according to the present invention and/or a host transformed with a vector comprising a nucleic acid molecule comprising a nucleotide sequence encoding a polypeptide according to the present invention in a method for the treatment and/or prevention of a disorder, disease or condition associated with Gram-negative bacteria. In particular the disorder, disease or condition may be caused by Gram-negative bacteria of bacterial groups, families, genera or species comprising strains pathogenic for humans or animals like *Enterobacteriaceae* (Escherichia, especially *E. coli, Salmonella, Shigella, Citrobacter, Edwardsiella, Enterobacter, Hafnia, Klebsiella,* especially *K. pneumoniae, Morganella, Proteus, Providencia, Serratia, Yersinia*), *Pseudomonadaceae* (*Pseudomonas,* especially *P. aeruginosa*), *Burkholderia, Stenotrophomonas, Shewanella, Sphingomonas, Comamonas, Neisseria, Moraxella, Vibrio, Aeromonas, Brucella, Francisella, Bordetella, Legionella, Bartonella, Coxiella, Haemophilus, Pasteurella, Mannheimia, Actinobacillus, Gardnerella, Spirochaetaceae* (*Treponema* and *Borrelia), Leptospiraceae, Campylobacter, Helicobacter, Spirillum, Streptobacillus, Bacteroidaceae (Bacteroides, Fusobacterium, Prevotella, Porphyromonas), Acinetobacter,* especially *A. baumannii.* In particular, the treatment and/or prevention of the disorder, disease or condition may be caused by *Pseudomonas aeruginosa, Pseudomonas putida, Burkholderia pseudomallei,* E. coli and/or *Salmonella* Typhimurium.

In a further aspect the present invention relates to a method of treating a disorder, disease or condition in a subject in need of treatment and/or prevention, which method comprises administering to said subject an effective amount of a polypeptide according to the present invention and/or an effective amount of a host transformed with a nucleic acid comprising a nucleotide sequence encoding a polypeptide according to the present invention or an effective amount of a composition according to the present invention. The subject may for example be a human or an animal. In particular said method of treatment may be for the treatment and/or prevention of infections of the skin, of soft tissues, the respiratory system, the lung, the digestive tract, the eye, the ear, the teeth, the nasopharynx, the mouth, the bones, the vagina, of wounds of bacteraemia and/or endocarditis caused by Gram-negative bacteria, in particular by the Gram-negative bacteria as listed above.

The dosage and route of administration used in a method of treatment (or prophylaxis) according to the present invention depends on the specific disease/site of infection to be treated. The route of administration may be for example oral, topical, nasopharyngeal, parenteral, intravenous, rectal or any other route of administration. For application of a polypeptide according to the present invention and/or an effective amount of a host transformed with a nucleic acid comprising a nucleotide sequence encoding a polypeptide according to the present invention or a composition according to the present invention to a site of infection (or site endangered to be infected) a formulation may be used that protects the active compounds from environmental influences such as proteases, oxidation, immune response etc., until it reaches the site of infection. Therefore, the formulation may be capsule, dragee, pill, suppository, injectable solution or any other medical reasonable galenic formulation. Preferably, the galenic formulation may comprise suitable carriers, stabilizers, flavourings, buffers or other suitable reagents. For example, for topical application the formulation may be a lotion or plaster, for nasopharyngeal application the formulation may be saline solution to be applied via a spray to the nose.

Preferably, a polypeptide according to the present invention is used for medical treatment, if the infection to be treated (or prevented) is caused by multiresistant bacterial strains, in particular by strains resistant against one or more of the following antibiotics: streptomycin, tetracycline, cephalothin, gentamicin, cefotaxime, cephalosporin, ceftazidime or imipenem. Furthermore, a polypeptide according to the present invention can be used in methods of treatment by administering it in combination with conventional antibacterial agents, such as antibiotics, lantibiotics, bacteriocins or endolysins, etc.

The present invention also relates to a pharmaceutical pack comprising one or more compartments, wherein at least one compartment comprises one or more polypeptides according to the present invention, one or more hosts transformed with a nucleic acid comprising a nucleotide sequence encoding a polypeptide according to the present invention and/or a composition according to the present invention.

In another aspect the present invention relates to a process of preparation of a pharmaceutical composition, said process comprising admixing one or more polypeptides according to the present invention and/or one or more hosts transformed with a nucleic acid comprising a nucleotide sequence encoding a polypeptide according to the present invention with a pharmaceutically acceptable diluent, excipient or carrier.

In an even further aspect the composition according to the present invention is a cosmetic composition. Several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of the polypeptide according to the present invention in order to degrade already existing or freshly settling pathogenic Gram-negative bacteria.

In a further aspect the present invention relates to a polypeptide according to the present invention for use as diagnostic means in medicinal diagnostics, food diagnostics, feed diagnostics or environmental diagnostics, in particular as a diagnostic means for the diagnostic of bacterial infection or contamination caused in particular by Gram-negative bacteria. In this respect the polypeptide according to the present invention may be used as a tool to specifically degrade pathogenic bacteria, in particular Gram-negative pathogenic bacteria. The degradation of the bacterial cells by the polypeptide according to the present invention can be supported by the addition of detergents like Triton X-100 or other additives which weaken the bacterial cell envelope like polymyxin B. Specific cell degradation is needed as an initial step for subsequent specific detection of bacteria using nucleic acid based methods like PCR, nucleic acid hybridization or NASBA (Nucleic Acid Sequence Based Amplification), immunological methods like IMS, immunofluorescence or ELISA techniques, or other methods relying on the cellular content of the bacterial cells like enzymatic assays using proteins specific for distinct bacterial groups or species (e.g. β-galactosidase for enterobacteria, coagulase for coagulase positive strains).

In a further aspect the present invention relates to the use of the polypeptide according to the present invention for the treatment or prevention of Gram-negative bacterial contamination of foodstuff, of food processing equipment, of food processing plants, of (inanimate) surfaces coming into contact with foodstuff such as shelves and food deposit areas and in all other situations, where pathogenic, facultative pathogenic or other undesirable bacteria can potentially infest food material. Likewise, the polypeptide according to the present invention may be used for the treatment or prevention of Gram-negative bacterial contamination of feedstuff, of feed processing equipment, of feed processing plants, of (inanimate) surfaces coming into contact with feedstuff such as shelves and feed deposit areas and in all other situations, where pathogenic, facultative pathogenic or other undesirable bacteria can potentially infest feed material. The polypeptide according to the present invention may also be used for the treatment or prevention of Gram-negative bacterial contamination of medical devices and of all kinds of (inanimate) surfaces in hospitals and doctor's offices.

In particular, a polypeptide of the present invention may be used prophylactically as sanitizing agent. Said sanitizing agent may be used before or after surgery, or for example during hemodialysis. Moreover, premature infants and immunocompromised persons, or those subjects with need for prosthetic devices may be treated with a polypeptide according to the present invention. Said treatment may be either prophylactically or during acute infection. In the same context, nosocomial infections, especially by antibiotic resistant strains like *Pseudomonas aeruginosa* (FQRP), *Acinetobacter* species and *Enterobacteriaceae* such as *E.coli, Salmonella, Shigella, Citrobacter, Edwardsiella, Enterobacter, Hafnia, Klebsiella, Morganella, Proteus, Providencia, Serratia* and *Yersinia* species may be treated prophylactically or during acute phase with a polypeptide of the present invention. Therefore, a polypeptide according to the present invention may be used as a disinfectant also in combination with other ingredients useful in a disinfecting solution like detergents, tensids, solvents, antibiotics, lantibiotics, or bacteriocins.

The following examples explain the present invention but are not considered to be limiting to the disclosure or the appended claims. Unless indicated differently, molecular biological standard methods were used, as e.g., described by Sambrock et al., 1989, Molecular Cloning: A Laboratory Manual, 2nd edition, Cold Spring Harbor Laboratory Press, Cold 15 Spring Harbor, New York.

### EXAMPLE 1. Cloning, expression and purification of the endolysin CkP1gp131 of the Citrobacter koseri phage CkP1

CkP1gp131 (164 amino acids long, MW = 18609 Da; SEQ ID No. 1) is a globular endolysin originating from *Citrobacter koseri* phage CkP1 predicted to possess a lysozyme-like superfamily catalytic domain (from amino acids 1 to 163; SEQ ID No. 3) (see Figure 1). Purified genomic DNA of phage CkP1 was used as a template for the amplification of the open reading frame ORF131 encoding the endolysin CkP1gp131 in a standard PCR reaction with KAPA HiFi polymerase (Kapa Biosystems) using the following PCR parameters:
Start: 95°C, 2min; followed by 35 cycles of [98°C (20 sec) → 60°C (15 sec) → 72 °C (20 sec)], followed by 72°C (5 min) & cooling to 4°C & storage.

Primers used during standard PCR amplification of ORF131 encoding endolysin CkP1gp131 were:
ORF131 forward primer 5' gggCATATGAACATTTTTAAAATGCTTCG 3' (SEQ ID No. 9)
ORF131 reverse primer 5' gggGGATCCTCATTTATATGCGTTCCATG 3' (SEQ ID No. 10)

The obtained PCR fragment was then ligated in the commercial available pET28a expression vector (Novagen). The obtained amino acid & DNA sequences for the recombinant CkP1gp131 are listed in SEQ ID No. 7 and SEQ ID No. 8, respectively.

Recombinant expression of CkP1gp131 was performed in exponentially growing *E. coli* BL21 (λDE3) cells after induction with 0.5 mM IPTG (isopropylthiogalactoside) at 37°C for a period of 4 hours. The endolysin was then applied to Ni²⁺-NTA resin stacked in HisTrapTM HP 1 ml columns (GE Healthcare) for purification, using the N-terminal 6xHis-tag, encoded by the pET28a expression vector. The purification process envolved four steps:
1. Equilibrium step - 10 mL of washing buffer (25 mM imidazole, 0.5 mM NaCl and 20 mM NaH₂P0₄ - NaOH on pH 7.4 concentration).
2. Loading step - Loading of total soluble expressed *E. coli* extract that was resuspended in washing buffer (25 mM imidazole, 0.5 mM NaCl and 20 mM NaH₂P0₄ - NaOH on pH 7.4 concentration).
3. Washing step - 10 mL of washing buffer (25 mM imidazole, 0.5 mM NaCl and 20 mM NaH₂P0₄ - NaOH on pH 7.4 concentration).
4. Elution step - 2 mL of elution buffer (250 mM imidazole, 0.5 mM NaCl and 20 mM NaH₂P0₄ - NaOH on pH 7.4 concentration).

Eluted protein fractions of CkP1gp131 (MW = 20772 Da) were visualized by standard denaturation SDS-PAGE gel (Figure 2), achieving purities superior than 95%. Proteins were dialyzed in 10 mM HEPES at pH 7.0 (using Maxi GeBAflex-tube Dialysis Kit - Gene Bio-Application L.T.D) and concentration determined by the BCA™ Protein Assay Kit with bovine serum albumin as standard (Thermo Scientific). An expression yield of 37.2 mg per liter E. *coli* expression culture was obtained.

### EXAMPLE 2. Antibacterial effect of Citrobacter koseri phage CKP1 endolysin (SEQ ID No. 7) against a panel of Gram-negative pathogenic bacteria

For the evaluation of the antibacterial effect, all antibacterial experiments were performed using 50 µg/ml of recombinant CkP1gp131 (SEQ ID No. 7) against mid-exponential phase cells in 10 mM HEPES (pH of 7.0) and incubated for 37°C for 2 hours (Figure 3).

The relative inactivation in logarithmic units (= log₁₀ (N₀/Nᵢ) with N₀ = number of untreated cells (in the negative control) and Nᵢ = number of treated cells counted after incubation) is used to quantify the antibacterial activity. Averages ± standard deviations are given for n = 4 repeats.

Using the upper formula, CkP1gp131 possesses an antibacterial activity against all tested Gram-negative bacteria, ranging from 1 up to >5 logs (reaching the limit of detection of <100 cfu/ml).

### EXAMPLE 3. Antibacterial effect of Citrobacter koseri phage CKP1 endolysin (SEQ ID No. 7) against Citrobacter koseri under various pH conditions

Exponentially growing *C. koseri* cells (≈3x10⁹ CFU/ml) were 100x diluted into 20 mM of different buffer systems: Sodium citrate (pH 6); HEPES (pH 7-8) and Boric acid (pH 9). Reaction started by mixing 50 µL of cells with 50 µL of recombinant endolysin (or Hepes buffer as negative control) in order to have 5 or 50 µg/ml end concentration that. After incubation for 2 hours, the antibacterial effect was assessed by quantification of the number of CFUs and expressed as relative inactivation in logarithmic units (= log10 (N₀/Nᵢ) with N₀ = number of untreated cells and Nᵢ = number of cells after treatment).

### EXAMPLE 4. Antibacterial effect of Citrobacter koseri phage CKP1 endolysin (SEQ ID No. 7) against Citrobacter koseri under various ionic strength conditions

Exponentially growing *C. koseri* cells (≈3x10⁹ CFU/ml) were 100x diluted into 20 mM of HEPES pH 7 and were incubated with 5 or 50 µg/ml of endolysin (end concentration) for 2 hours, with increasing amounts of NaCl (0-500 mM). After incubation for 2 hours, the antibacterial effect was assessed by quantification of the number of CFUs and expressed as relative inactivation in logarithmic units (= log10 (N₀/Nᵢ) with N₀ = number of untreated cells and Nᵢ = number of cells after treatment).

### EXAMPLE 5. Antibacterial effect of Citrobacter koseri phage CKP1 endolysin (SEQ ID No. 7) against Citrobacter koseri under various temperature conditions

Exponentially growing *C. koseri* cells were incubated with the endolysin using 20 mM of HEPES buffer, pH 7.0; 0 mM NaCl. After incubation for 2 hours, the antibacterial effect was assessed by quantification of the number of CFUs and expressed as relative inactivation in logarithmic units (= log10 (N₀/Nᵢ) with N₀ = number of untreated cells and Nᵢ = number of cells after treatment).

### EXAMPLE 6. Antibacterial effect of Citrobacter koseri phage CKP1 endolysin (SEQ ID No. 7) against Citrobacter koseri over time

The endolysin (SEQ ID No. 7) was kept at 37°C for several days in 20 mM Hepes pH 7.0 buffer, a) At day 0, 5 10, 20 and 30, 50 µg/ml of endolysin was a) tested against exponentially growing *C. koseri* cells (≈3x10⁷ CFU/ml) at 37°C and for 2 hours, using again HEPES buffer as a negative control. After incubation, the antibacterial effect was assessed by quantification of the number of CFUs and expressed as relative inactivation in logarithmic units (= log10 (N₀/Nᵢ) with N₀ = number of untreated cells and Nᵢ = number of cells after treatment).

At day 0, 5 10, 20 and 30, the same sample was analysed on circular dichroism spectra in the far- and near-UV region (190 to 260 nm). The endolysin demonstrated to be highly stable, as no significant secondary structure changes were observed. Therefore, the CD thermal stability analysis demonstrates that the endolysin activity is still present after one month at 37°C.

## Claims

1. A polypeptide comprising an amino acid sequence according to SEQ ID No. 1 or a fragment or derivative thereof, wherein the derivative is an amino acid sequence having a substitution of 1, 2, 3, 4, or 5 amino acids in the amino acid sequence of SEQ ID No. 1 and/or 3, and wherein the fragment comprises an amino acid sequence according to SEQ ID No. 3, SEQ ID No. 5 or SEQ ID No. 6.

2. The polypeptide according to claim 1, wherein the polypeptide is fused at the N- or C-terminus to a peptide stretch having membrane or LPS disrupting activity.

3. The polypeptide according to any one of the preceding claims comprising additionally a tag, preferably a His6-tag.

4. The polypeptide according to any one of the preceding claims, wherein the polypeptide has endolysin activity.

5. An isolated nucleic acid molecule encoding a polypeptide according to any one of claims 1 to 4.

6. A vector comprising the nucleic acid molecule according to claim 5.

7. A host cell comprising the nucleic acid molecule according to claim 5 or the vector according to claim 6.

8. The polypeptide according to any one of claims 1 to 4 for use in a method of treating the human or animal body by surgery or therapy and diagnostic methods practised on the human or animal body.

9. Use of an polypeptide according to any one of claims 1 to 4 as an antimicrobial in food, feed or in cosmetics, or as a disinfecting agent.

10. The polypeptide according to any one of claims 1 to 4 for use in a method for treatment or prevention of Gram-negative bacterial infections.

11. Use of the polypeptide according to any one of claims 1 to 4 for the treatment or prevention of Gram-negative bacterial contamination of foodstuff, of food processing equipment, of food processing plants, of surfaces coming into contact with foodstuff, of feed, of feed processing equipment, of feed processing plants, of surfaces coming into contact with feed, of medical devices, of surfaces in hospitals and doctor's offices.

12. Pharmaceutical composition comprising the polypeptide according to any one of claims 1 to 5 and a pharmaceutically acceptable diluent or excipient.

## Patentansprüche

1. Polypeptid, umfassend eine Aminosäuresequenz gemäß SEQ ID Nr. 1 oder ein Fragment oder Derivat davon, wobei das Derivat eine Aminosäuresequenz mit einer Substitution von 1, 2, 3, 4 oder 5 Aminosäuren in der Aminosäuresequenz von SEQ ID Nr. 1 und/oder 3 ist, und wobei das Fragment eine Aminosäuresequenz gemäß SEQ ID Nr. 3, SEQ ID Nr. 5 oder SEQ ID Nr. 6 umfasst.

2. Polypeptid nach Anspruch 1, wobei das Polypeptid am N- oder C-Terminus an einen Peptidabschnitt mit membran- oder LPS-zerstörender Aktivität fusioniert ist.

3. Polypeptid nach einem der vorhergehenden Ansprüche, umfassend zusätzlich ein Tag, vorzugsweise einen His6-Tag.

4. Polypeptid nach einem der vorhergehenden Ansprüche, wobei das Polypeptid Endolysin-Aktivität aufweist.

5. Isoliertes Nukleinsäuremolekül, das ein Polypeptid nach einem der Ansprüche 1 bis 4 kodiert.

6. Vektor, umfassend das Nukleinsäuremolekül nach Anspruch 5.

7. Wirtszelle, umfassend das Nukleinsäuremolekül nach Anspruch 5 oder den Vektor nach Anspruch 6.

8. Polypeptid nach einem der Ansprüche 1 bis 4 zur Verwendung in einem Verfahren zur Behandlung des menschlichen oder tierischen Körpers durch chirurgische oder therapeutische und diagnostische Verfahren, die am menschlichen oder tierischen Körper praktiziert werden.

9. Verwendung eines Polypeptids nach einem der Ansprüche 1 bis 4 als antimikrobielles Mittel in Lebens-, Futtermitteln oder in Kosmetika oder als Desinfektionsmittel.

10. Polypeptid nach einem der Ansprüche 1 bis 4 zur Verwendung in einem Verfahren zur Behandlung oder Prävention von gramnegativen bakteriellen Infektionen.

11. Verwendung des Polypeptids nach einem der Ansprüche 1 bis 4 zur Behandlung oder Vorbeugung einer gramnegativen bakteriellen Kontamination von Lebensmitteln, von Lebensmittelverarbeitungsgeräten, von Lebensmittelverarbeitungsanlagen, von Oberflächen, die mit Lebensmitteln in Kontakt kommen, von Futtermitteln, von Futtermittelverarbeitungsgeräten, von Futtermittelverarbeitungsanlagen, von Oberflächen, die mit Futtermitteln in Kontakt kommen, von Medizinprodukten, von Oberflächen in Krankenhäusern und Arztpraxen.

12. Pharmazeutische Zusammensetzung, umfassend das Polypeptid nach einem der Ansprüche 1 bis 5 und ein pharmazeutisch verträgliches Verdünnungsmittel oder Hilfststoff.

## Revendications

1. Polypeptide comprenant une séquence d'acides aminés selon la SEQ ID NO. 1 ou un fragment ou dérivé de celle-ci, dans lequel le dérivé est une séquence d'acides aminés ayant une substitution de 1, 2, 3, 4 ou 5 acides aminés dans la séquence d'acides aminés des SEQ ID NO : 1 et/ou 3, et dans lequel le fragment comprend une séquence d'acides aminés selon la SEQ ID NO : 3, la SEQ ID NO : 5 ou la SEQ ID NO. 6.

2. Polypeptide selon la revendication 1, ledit polypeptide étant fusionné à l'extrémité N- ou C-terminale à un segment peptidique ayant une activité de rupture de membrane ou de LPS.

3. Polypeptide selon l'une quelconque des revendications précédentes, comprenant en outre une étiquette, de préférence une étiquette His6.

4. Polypeptide selon l'une quelconque des revendications précédentes, ledit polypeptide ayant une activité d'endolysine.

5. Molécule d'acide nucléique isolée codant un polypeptide selon l'une quelconque des revendications 1 à 4.

6. Vecteur comprenant la molécule d'acide nucléique selon la revendication 5.

7. Cellule hôte comprenant la molécule d'acide nucléique selon la revendication 5 ou le vecteur selon la revendication 6.

8. Polypeptide selon l'une quelconque des revendications 1 à 4 pour une utilisation dans une méthode de traitement chirurgical ou thérapeutique du corps humain ou animal et dans des méthodes diagnostiques appliquées au corps humain ou animal.

9. Utilisation d'un polypeptide selon l'une quelconque des revendications 1 à 4 en tant qu'un agent antimicrobien dans un aliment, un aliment pour animaux ou un cosmétique, ou en tant qu'un agent désinfectant.

10. Polypeptide selon l'une quelconque des revendications 1 à 4 pour une utilisation dans une méthode de traitement ou de prévention d'infections bactériennes Gram négatives.

11. Utilisation du polypeptide selon l'une quelconque des revendications 1 à 4 pour le traitement ou la prévention d'une contamination bactérienne Gram négative d'un aliment, d'un équipement de transformation des aliments, ou d'usines de transformation des aliments, de surfaces venant en contact avec des aliments, d'un aliment pour animaux, d'un équipement de transformation d'aliments pour animaux, d'usines de transformation d'aliments pour animaux, de surfaces venant en contact avec des aliments pour animaux, de dispositifs médicaux, de surfaces dans des hôpitaux et des cabinets médicaux.

12. Composition pharmaceutique comprenant le polypeptide selon l'une quelconque des revendications 1 à 5 et un diluant ou excipient pharmaceutiquement acceptable.


### SEQUENCE LISTING

<110> UNIVERSIDADE DO MINHO
<120> Novel endolysin
<130> UDM-001 PCT
<150> EP16204770.8
   <151> 2016-12-16
<160> 10
<170> PatentIn version 3.5
<210> 1
   <211> 164
   <212> PRT
   <213> Unknown
<220>
   <223> Endolysin derived from Citrobacter Koseri phage CkP1
<400> 1
   Met Asn Ile Phe Lys Met Leu Arg Ile Asp Glu Gly Tyr Asp Ser Lys
   1               5                   10                  15
   Ile Tyr Lys Asp Thr Glu Gly Phe Trp Thr Ile Gly Ile Gly His Leu
               20                  25                  30
   Leu Thr Arg Asp Pro Ser Leu Glu Val Ala Lys Arg Glu Leu Asp Lys
           35                  40                  45
   Leu Val Gly Arg Lys Cys Asn Gly Gln Ile Thr Gln Ser Glu Ala Glu
       50                  55                  60
   Lys Ile Phe Ala Asp Asp Val Asp Lys Ala Ile Asn Gly Ile Lys Lys
   65                  70                  75                  80
   Asn Ala Ser Leu Lys Pro Val Tyr Asp Ser Leu Asp Gly Asp Asp Pro
                   85                  90                  95
   Arg Gln Ala Ala Leu Ile Asn Met Val Phe Gln Met Gly Val Ala Gly
               100                 105                 110
   Val Ala Gly Phe Thr Asn Ser Met Arg Met Val Lys Glu Lys Arg Trp
           115                 120                 125
   Ala Asp Ala Ala Val Asn Leu Ala Gln Ser Lys Trp Tyr Arg Gln Thr
       130                 135                 140
   Pro Asn Arg Ala Lys Arg Val Ile Glu Thr Phe Arg Thr Gly Thr Trp
   145                 150                 155                 160
   Asn Ala Tyr Lys
<210> 2
   <211> 492
   <212> DNA
   <213> Unknown
<220>
   <223> Endolysin derived from Citrobacter Koseri phage CkP1
<400> 2
   atgaacattt ttaaaatgct tcgtatcgat gaaggatacg attctaaaat ctataaagat        60
   accgaagggt tttggactat cggtatcggt caccttttaa ctcgcgaccc ttctcttgaa       120
   gtagccaaaa gagaacttga taaactcgtt gggcgtaagt gcaacggtca aatcactcag       180
   tcggaagcag aaaagatttt cgctgatgat gttgataaag ctattaatgg aatcaagaaa       240
   aacgcttctc tgaaaccggt ttatgattct cttgatggag atgatccgcg tcaagctgca       300
   ctcatcaata tggtatttca aatgggtgta gctggtgtag ctggcttcac taattctatg       360
   agaatggtaa aagaaaaacg ttgggctgat gcagctgtaa atttagctca atcaaaatgg       420
   tatcgtcaaa ctccgaatcg cgctaaacga gtaattgaaa cattccgcac tggaacatgg       480
   aacgcatata aa                                                           492
<210> 3
   <211> 163
   <212> PRT
   <213> Unknown
<220>
   <223> Catalytic domain of endolysin derived from Citrobacter Koseri phage CkP1
<400> 3
   Met Asn Ile Phe Lys Met Leu Arg Ile Asp Glu Gly Tyr Asp Ser Lys
   1               5                   10                  15
   Ile Tyr Lys Asp Thr Glu Gly Phe Trp Thr Ile Gly Ile Gly His Leu
               20                  25                  30
   Leu Thr Arg Asp Pro Ser Leu Glu Val Ala Lys Arg Glu Leu Asp Lys
           35                  40                  45
   Leu Val Gly Arg Lys Cys Asn Gly Gln Ile Thr Gln Ser Glu Ala Glu
       50                  55                  60
   Lys Ile Phe Ala Asp Asp Val Asp Lys Ala Ile Asn Gly Ile Lys Lys
   65                  70                  75                  80
   Asn Ala Ser Leu Lys Pro Val Tyr Asp Ser Leu Asp Gly Asp Asp Pro
   85 90 95
   Arg Gln Ala Ala Leu Ile Asn Met Val Phe Gln Met Gly Val Ala Gly
   100 105 110
   Val Ala Gly Phe Thr Asn Ser Met Arg Met Val Lys Glu Lys Arg Trp
   115 120 125
   Ala Asp Ala Ala Val Asn Leu Ala Gln Ser Lys Trp Tyr Arg Gln Thr
   130 135 140
   Pro Asn Arg Ala Lys Arg Val Ile Glu Thr Phe Arg Thr Gly Thr Trp
   145 150 155 160
   Asn Ala Tyr
<210> 4
   <211> 489
   <212> DNA
   <213> Unknown
<220>
   <223> Catalytic domain of endolysin derived from Citrobacter Koseri phage CkP1
<400> 4
   atgaacattt ttaaaatgct tcgtatcgat gaaggatacg attctaaaat ctataaagat 60
   accgaagggt tttggactat cggtatcggt caccttttaa ctcgcgaccc ttctcttgaa 120
   gtagccaaaa gagaacttga taaactcgtt gggcgtaagt gcaacggtca aatcactcag 180
   tcggaagcag aaaagatttt cgctgatgat gttgataaag ctattaatgg aatcaagaaa 240
   aacgcttctc tgaaaccggt ttatgattct cttgatggag atgatccgcg tcaagctgca 300
   ctcatcaata tggtatttca aatgggtgta gctggtgtag ctggcttcac taattctatg 360
   agaatggtaa aagaaaaacg ttgggctgat gcagctgtaa atttagctca atcaaaatgg 420
   tatcgtcaaa ctccgaatcg cgctaaacga gtaattgaaa cattccgcac tggaacatgg 480
   aacgcatat 489
<210> 5
   <211> 163
   <212> PRT
   <213> Unknown
<220>
   <223> Endolysin derived from Citrobacter Koseri phage CkP1 w/o N-terminal methionine
<400> 5
   Asn Ile Phe Lys Met Leu Arg Ile Asp Glu Gly Tyr Asp Ser Lys Ile
   1               5                   10                  15
   Tyr Lys Asp Thr Glu Gly Phe Trp Thr Ile Gly Ile Gly His Leu Leu
               20                  25                  30
   Thr Arg Asp Pro Ser Leu Glu Val Ala Lys Arg Glu Leu Asp Lys Leu
           35                  40                  45
   Val Gly Arg Lys Cys Asn Gly Gln Ile Thr Gln Ser Glu Ala Glu Lys
       50                  55                  60
   Ile Phe Ala Asp Asp Val Asp Lys Ala Ile Asn Gly Ile Lys Lys Asn
   65                  70                  75                  80
   Ala Ser Leu Lys Pro Val Tyr Asp Ser Leu Asp Gly Asp Asp Pro Arg
                   85                  90                  95
   Gln Ala Ala Leu Ile Asn Met Val Phe Gln Met Gly Val Ala Gly Val
               100                 105                 110
   Ala Gly Phe Thr Asn Ser Met Arg Met Val Lys Glu Lys Arg Trp Ala
           115                 120                 125
   Asp Ala Ala Val Asn Leu Ala Gln Ser Lys Trp Tyr Arg Gln Thr Pro
       130                 135                 140
   Asn Arg Ala Lys Arg Val Ile Glu Thr Phe Arg Thr Gly Thr Trp Asn
   145                 150                 155                 160
   Ala Tyr Lys
<210> 6
   <211> 162
   <212> PRT
   <213> Unknown
<220>
   <223> Catalytic domain of endolysin derived from Citrobacter Koseri phage CkP1 w/o N-terminal methionine
<400> 6
   Asn Ile Phe Lys Met Leu Arg Ile Asp Glu Gly Tyr Asp Ser Lys Ile
   1               5                   10                  15
   Tyr Lys Asp Thr Glu Gly Phe Trp Thr Ile Gly Ile Gly His Leu Leu
   20 25 30
   Thr Arg Asp Pro Ser Leu Glu Val Ala Lys Arg Glu Leu Asp Lys Leu
   35 40 45
   Val Gly Arg Lys Cys Asn Gly Gln Ile Thr Gln Ser Glu Ala Glu Lys
   50 55 60
   Ile Phe Ala Asp Asp Val Asp Lys Ala Ile Asn Gly Ile Lys Lys Asn
   65 70 75 80
   Ala Ser Leu Lys Pro Val Tyr Asp Ser Leu Asp Gly Asp Asp Pro Arg
   85 90 95
   Gln Ala Ala Leu Ile Asn Met Val Phe Gln Met Gly Val Ala Gly Val
   100 105 110
   Ala Gly Phe Thr Asn Ser Met Arg Met Val Lys Glu Lys Arg Trp Ala
   115 120 125
   Asp Ala Ala Val Asn Leu Ala Gln Ser Lys Trp Tyr Arg Gln Thr Pro
   130 135 140
   Asn Arg Ala Lys Arg Val Ile Glu Thr Phe Arg Thr Gly Thr Trp Asn
   145 150 155 160
   Ala Tyr
<210> 7
   <211> 184
   <212> PRT
   <213> Artificial sequence
<220>
   <223> Recombninant endolysin derived from Citrobacter Koseri phage CkP1 including N-terminal HisTag
<400> 7
   Met Gly Ser Ser His His His His His His Ser Ser Gly Leu Val Pro
   1 5 10 15
   Arg Gly Ser His Met Asn Ile Phe Lys Met Leu Arg Ile Asp Glu Gly
   20 25 30
   Tyr Asp Ser Lys Ile Tyr Lys Asp Thr Glu Gly Phe Trp Thr Ile Gly
   35 40 45
   Ile Gly His Leu Leu Thr Arg Asp Pro Ser Leu Glu Val Ala Lys Arg
           50                  55                  60
   Glu Leu Asp Lys Leu Val Gly Arg Lys Cys Asn Gly Gln Ile Thr Gln
   65                  70                  75                  80
   Ser Glu Ala Glu Lys Ile Phe Ala Asp Asp Val Asp Lys Ala Ile Asn
                   85                  90                  95
   Gly Ile Lys Lys Asn Ala Ser Leu Lys Pro Val Tyr Asp Ser Leu Asp
               100                 105                 110
   Gly Asp Asp Pro Arg Gln Ala Ala Leu Ile Asn Met Val Phe Gln Met
           115                 120                 125
   Gly Val Ala Gly Val Ala Gly Phe Thr Asn Ser Met Arg Met Val Lys
       130                 135                 140
   Glu Lys Arg Trp Ala Asp Ala Ala Val Asn Leu Ala Gln Ser Lys Trp
   145                 150                 155                 160
   Tyr Arg Gln Thr Pro Asn Arg Ala Lys Arg Val Ile Glu Thr Phe Arg
                   165                 170                 175
   Thr Gly Thr Trp Asn Ala Tyr Lys
               180
<210> 8
   <211> 552
   <212> DNA
   <213> Artificial sequence
<220>
   <223> Recombninant endolysin derived from Citrobacter Koseri phage CkP1 including N-terminal HisTag
<400> 8
   atgggcagca gccatcatca tcatcatcac agcagcggcc tggtgccgcg cggcagccat    60
   atgaacattt ttaaaatgct tcgtatcgat gaaggatacg attctaaaat ctataaagat   120
   accgaagggt tttggactat cggtatcggt caccttttaa ctcgcgaccc ttctcttgaa   180
   gtagccaaaa gagaacttga taaactcgtt gggcgtaagt gcaacggtca aatcactcag   240
   tcggaagcag aaaagatttt cgctgatgat gttgataaag ctattaatgg aatcaagaaa   300
   aacgcttctc tgaaaccggt ttatgattct cttgatggag atgatccgcg tcaagctgca   360
   ctcatcaata tggtatttca aatgggtgta gctggtgtag ctggcttcac taattctatg   420
   agaatggtaa aagaaaaacg ttgggctgat gcagctgtaa atttagctca atcaaaatgg   480
   tatcgtcaaa ctccgaatcg cgctaaacga gtaattgaaa cattccgcac tggaacatgg   540
   aacgcatata aa                                                        552
<210> 9
   <211> 29
   <212> DNA
   <213> Artificial sequence
<220>
   <223> ORF131 forward primer
<400> 9
   gggcatatga acatttttaa aatgcttcg 29
<210> 10
   <211> 29
   <212> DNA
   <213> Artificial sequence
<220>
   <223> ORF131 reverse primer
<400> 10
   gggggatcct catttatatg cgttccatg 29